Europäisches Patentamt

European Patent Office

Office européen des brevets

(11) Publication number: **0 397 446**
**A2**

## EUROPEAN PATENT APPLICATION

(21) Application number: **90304944.3**

(51) Int. Cl.5: **A61M 16/16**

(22) Date of filing: **08.05.90**

(30) Priority: **08.05.89 GB 8910554**

(43) Date of publication of application:
**14.11.90 Bulletin 90/46**

(84) Designated Contracting States:
**DE DK ES FR IT NL SE**

(71) Applicant: **RESPIRATORY SUPPORT PRODUCTS INC**
**3118 Alpine**
**Santa Ana, California 92704(US)**

(72) Inventor: **Beran, Anthony Vincent**
**1472 La Loma Drive**
**Santa Ana, California 92705(US)**

(74) Representative: **Flint, Jonathan McNeill**
**SMITHS INDUSTRIES PUBLIC LIMITED**
**COMPANY 765 Finchley Road**
**London NW11 8DS(GB)**

(54) **Humidification in respiratory systems.**

(57) A respiratory humidifier has an outer flexible, gas-tight bag 10 containing water 12 and an exothermic chemical heater 16. A sandwich of an open-celled foam 36 and a vapour-permeable/water impermeable layer 30 is secured on the inside of the bag by compressing and welding the sandwich to the wall of the bag to form a labyrinth path for gas through the foam. Water vapour in the bag permeates the foam so that respiratory gas is humidified without contacting the water.

Fig. 1

EP 0 397 446 A2

## HUMIDIFICATION IN RESPIRATORY SYSTEMS

This invention relates to humidifiers for humidifying respiratory gas.

Systems for delivering respiratory gas to patients include apparatus which serves as the source of air, oxygen and sometimes other respiratory gases, in appropriate proportion and at an appropriate positive pressure. The gas is delivered to the patient through a delivery conduit to what is sometimes called a "patient connection". The patient connection may be a mask or an intubation set, an incubator or even a tent. Particularly when the purpose of the respiratory system includes forced or paced ventilation of the patient, the system will include an exhaust conduit which extends from the patient connection to a point of negative gauge pressure.

In many cases, it is required that the gas be delivered to the patient connection at an elevated temperature near to that of the body and that the gas be humid to the point of saturation or nearly so. Heating and humidifying are readily accomplished, however, to do so in a way that minimizes discomfort and danger to the patient is seldom easy. The problem is twofold. The delivered gas must not exceed a safe temperature, and no water (either humidifying water or condensate) must reach the patient. If humidification of the gas is accomplished at the supply apparatus or in the supply conduit at an appreciable distance from the patient connection, an excessive amount of condensate will form in the delivery conduit as the gas passes along the conduit and is cooled. If that condensate reaches the patient's mask or intubation set, the patient, an infant or comatose patient for example, may be drowned.

Temperature measurement and control are accomplished automatically with relative ease. Heating is done electrically and is readily controlled electronically. Humidification is less convenient. The conventional system employs a "bubbler", that is a container of water connected in the gas delivery conduit such that the gas must flow through the water. Gas from the source is conducted to a point in the bubbler below the level of the water, where it emerges into the water and bubbles up to the ullage space above the water. From the ullage space under pressure, it flows to the patient connection, still under pressure. Heating is accomplished at the humidifier or upstream from it, because heating it downstream would reduce the degree of humidification. Moving the humidifier downstream would reduce the opportunity for condensate to form but it would also increase the danger that a spill in the humidifier would reach and drown the patient. In practice, the distance from the gas source to the patient connection differs from case to case, patients must be free to move or be moved and, for additional reasons, the conduits, humidifier and heater are often assembled into jury rigged, temporary flow circuits. The danger that a humidifier or water trap will be dislodged from its mounting and spill water to the patient side of the device is a continuing danger.

Bubbler humidifiers also have the disadvantage that they require the use of sterile water because the gas flowpath is through the water. Bubbler humidifiers are also noisy which can interfere with sleep, be irritating and stressful to sedated patients.

It is an object of the invention to provide a humidifier which can be used to alleviate these disadvantages.

According to one aspect of the present invention there is provided a humidifier for humidifying respiratory gas, characterised in that the humidifier includes a porous member having a front wall and a rear wall bonded at a marginal bond, at least one of the walls being permeable to water vapour and impermeable to water, that there is a gas flow path through the porous member, and an intermediate water vapour porous foam layer within the porous member, and that the porous member is located within a gas-tight container at least partially filled with water such that gas flowing through the porous member is humidified by water vapour transmitted through the vapour permeable wall.

The container is preferably a flexible bag comprising a front wall and a rear wall of pliable material bonded together at a marginal bond. The container is preferably of a transparent plastics material. One wall of the porous member may be secured to a wall of the container. The container may have a sealable inlet. The porous member and the porous foam layer contained therein are preferably compressed and welded to form a barrier against the flow of gas through the foam from one portion of the member to another. The gas flow path is preferably formed at least in part by the welded barrier which defines a labyrinth through which respiratory gas flows. The porous member may be flexible and the foam layer may be of open celled polyurethane.

The humidifier preferably includes heating means which may be provided by an exothermic chemical reaction.

A humidifier in accordance with the present invention, will now be described, by way of example, with reference to the accompanying drawings, in which:

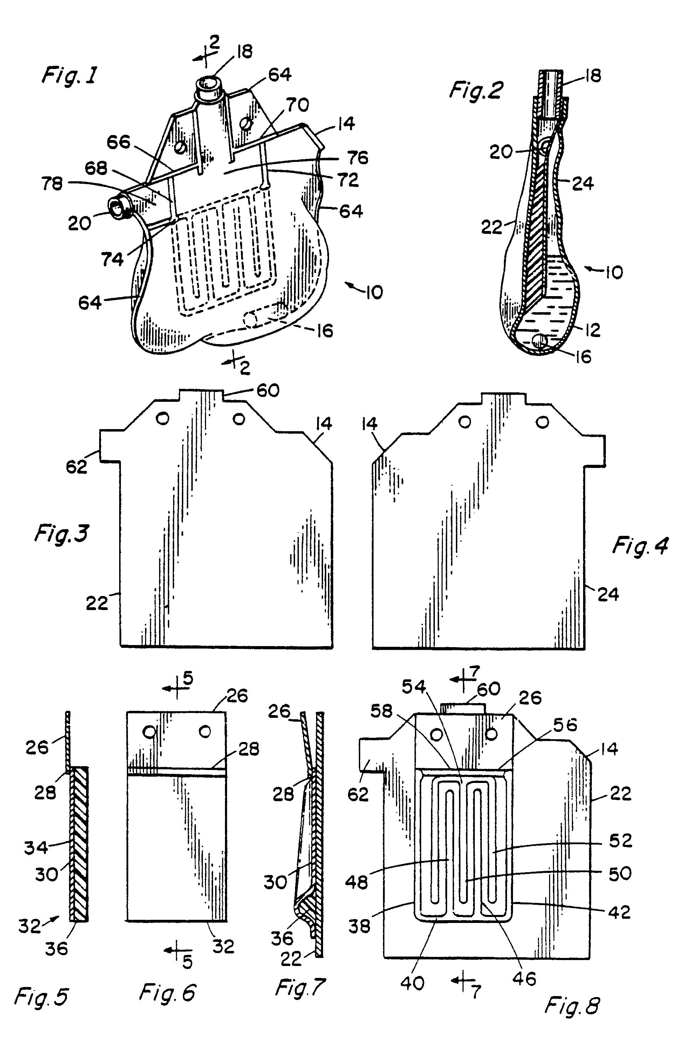

Figure 1 is a perspective view of the humidifier;

Figure 2 is a side elevation view along the line 2 - 2;

Figure 3 and 4 show front and rear walls of the container of the humidifer;

Figure 5 is a side elevation view of a part of the humidifier at a preliminary stage of manufacture;

Figure 6 is a front elevation view of the part in Figure 5;

Figure 7 is a side elevation of the part shown in Figures 5 and 6 at a later stage of manufacture; and

Figure 8 is a front elevation view of the part shown in Figure 7 assembled onto the front wall of the container.

The humidifier 10 has an inlet tube 18 and an outlet tube 20 for respiratory gases. Gas flows through a porous member 32 which is partly immersed in water 12 within an outer container.

The outer container comprises a front wall 22 and a rear wall 24 which are both of a pliable, transparent plastics material welded together about a marginal edge portion 64. The walls 22 and 24 are of a material that is substantially impermeable to both water and gas, that is, the walls are gastight and may, for example, be of PVC. The walls 22 and 24 are generally rectangular in shape, having a neck 60 at their upper end, which is secured with the inlet tube 18, and a neck 62 at one side, which is secured with the outlet tube 20. The welded seal extends around the periphery of the container except for a sealable opening 14 towards the upper end of the container which may be a press-to-close or zip lock type of construction. The peripheral weld also serves to secure the inlet and outlet tubes 18 and 20 into the humidifier.

The porous member 32 comprises an upper rectangular sheet 26 of PVC the lower edge 28 of which is bonded to the upper edge of the rear wall or layer 30 of a foam sandwich 34. The foam sandwich 34 has an intermediate layer 36 of an open celled resilient polyurethane or vinyl foam which is bonded on one side to the rear layer 30 and on the other side to the front wall 22 of the outer container which also forms the front wall of the porous member. The rear layer 30 comprises a layer of porous polytetrafluorethylene bonded to the foam 36 on one side and covered by a layer of porous polyester support material on its other side. The rear layer 30 is thereby gas permeable, permitting water vapour to flow through it readily but is impermeable to liquid water and to bacteria. The rear layer 30 is exposed to the interior of the container 10.

The foam sandwich 34 is formed into a labyrinth by compressing the foam 36 and applying bonding energy, such as by heating or ultrasonic welding, along weld lines. Weld lines 38, 40 and 42 along the side and bottom edge of the foam sandwich 34 form barriers to gas flow out of the edges of the porous member. Two vertical weld lines 46 and 48 extend upwardly from the bottom weld line 40. Three vertical weld lines 44, 50 and 52 of an M-Shape weld 54 extend downwardly being interdigitated between the upwardly directed welds 46 and 48. The weld lines 38 to 54 also serve to bond the porous member 32 to the front wall 22. The upwardly extending weld lines 46 and 48 terminate short of the upper end of the M-shape weld 54, whereas the downwardly extending weld lines 44, 50 and 52 terminate above the bottom weld line 40. In this way, the weld lines 46, 48, 44, 50 and 52 form barriers against the flow of gas between the left-hand and right-hand portions of the porous member 32, compelling gas to flow along a tortuous, labyrinth pathway having six parallel, vertical paths between the weld lines which interconnect with each other at five points where the flow direction is reversed. The upper end of the path between the two weld lines 42 and 52 provides the inlet 56 of the labyrinth; and the upper end of the path between the weld lines 38 and 44 provides the outlet 58. Flow of gas from the inlet tube 20 is directed to the inlet 56, and flow of gas out of the porous member 32 is directed from the outlet 58 to the outlet tube 20 by means of weld lines 66, 68, 70, 72 and 74 via portions 76 and 78 of the container.

All the materials used in the construction of the humidifier are medically compatible.

In operation, water is poured into the container 10 through the opening 14. Because there is no contact between the respiratory gas and the liquid water, there is no need for the water to be sterile; ordinary tap water can be used. When connected into a respiratory system, gas flows from the inlet tube 18 to the outlet tube 20 via the porous member 32. Because the rear wall 30 of the porous member 32 is permeable to water vapour, it allows water vapour from the interior of the outer container to flow into the foam passageway but prevents the entry of liquid water. Respiratory gas from the inlet 18 can also flow out through the rear wall 30 into the container 10. The walls 22 and 24 of the container are gas-tight, so gas flows from the porous member 32 into the ullage space of the container until the partial pressure of respiratory gas outside the porous member is equal to that inside. When this occurs, no more respiratory gas escapes.

The humidifier preferably also includes some means for heating the water in the container. This may take the form of a packet 16 containing a chemical which reacts exothermally with water, that is, it releases heat when added to water. The packet 16 is loose within the container so that it moves with the water to the lowest part of the container regardless of its orientation. The chemicals in the packet 16 are selected so that no substance is produced which is both harmful and capable of penetrating the respiratory gas flow path. An exothermic chemical heater has advantages over electrical heaters in that heating ceases if there is no water present thereby reducing the risk of overheating. It also avoids the need for external power connection.

There are many advantages to the humidifier of the present invention. The fact that ordinary tap water can be used makes the humidifier particularly useful in emergency situations and in locations where sterile water is not available. It also avoids the need to store and transport sterile water.

The humidifier is substantially soundless compared with bubbler humidifiers which makes its use less intrusive. There is also a reduced risk of water entering the respiratory gas flow path.

The humidifier can be made inexpensively so that it can be disposed of after use by a single patient. This avoids the need to sterilize equipment before reuse and reduces the risk of cross-infection. The humidifier can be made of flexible materials of low weight so that it is easy to pack, transport and store. It also makes it less prone to breakage compared with previous humidifiers involving glass containers.

A further advantage of the humidifier is that the resistance to passage of gas through it is substantially constant and independent of the level of water in the humidifier. This is a particular advantage with infants where pressure differentials are very low or are required to change very rapidly.

The foam interlayer in the porous member gives it structural support, thereby enabling the walls of the porous member to be flexible and thin enough to be highly permeable to gas whilst having a flowpath of relatively large cross-sectional area which provides minimal resistance to gas flow along it.

Various modifications are possible to the humidifier. The porous member need not be secured to the front wall of the container but could be freely suspended with the container so that both the front and rear walls are exposed to water vapour in the ullage. Instead of using an exothermic chemical reaction, the water could be heated by, for example, an electric heating element immersed in the water in the container or outside the humidifier.

The gas flowpath through the porous member need not be a labyrinth, or it could be a labyrinth of a different shape, providing that the length of the gas permeable section of the flowpath is sufficient to achieve the desired degree of humidification.

## Claims

1. A humidifier for humidifying respiratory gas, characterised in that the humidifier includes a porous member (32) having a front wall (22) and a rear wall (30) bonded at a marginal bond (38, 40, 42) at least one of the walls (30) being permeable to water vapour and impermeable to water, that there is a gas flow path through the porous member, and an intermediate water vapour porous foam layer (36) within the porous member, and that the porous member (32) is located within a gas-tight container (10) at least partially filled with water (12) such that gas flowing through the porous member is humidified by water vapour transmitted through the vapour permeable wall (30).

2. A humidifier according to Claim 1, characterised in that the container (10) is a flexible bag having a front wall (22) and a rear wall (24) of pliable material bonded together at a marginal bond (64).

3. A humidifier according to Claim 1 or 2, characterised in that the container (10) is of a transparent, plastics material.

4. A humidifier according to any of the preceding claims, characterised in that the porous member is secured to a wall (22) of the container (10).

5. A humidifier according to any one of the preceding claims, characterised in that the container (10) has a sealable inlet (14).

6. A humidifier according to any one of the preceding claims, characterised in that the porous member (32) and the porous foam layer (36) contained therein are compressed and welded to form a barrier (44, 48, 50, 46, 52) against the flow of gas through the foam from one portion of the member to another.

7. A humidifier according to Claim 6, characterised in that the gas flow path is formed at least in part by the welded barrier (44, 48, 50, 46, 52) which defines a labyrinth through which respiratory gas flows.

8. A humidifier according to any one of the preceding claims, characterised in that the porous member (32) is flexible.

9. A humidifier according to any one of the preceding claims, characterised in that the foam layer (36) is of open celled polyurethane.

10. A humidifier according to any one of the preceding claims, characterised in that the humidifier includes an exothermic chemical heater (16).

Fig.1

Fig.2

Fig.3

Fig.4

Fig.5

Fig.6

Fig.7

Fig.8